# EUROPEAN PATENT SPECIFICATION

(11) **EP 1 286 674 B1**
(45) Date of publication and mention of the grant of the patent: **09.07.2008**
(21) Application number: 01964398.0
(22) Date of filing: 24.08.2001
(51) Int. Cl.: A61K 31/375

(54) **COMPOSITION FOR POTENTIATING CANCER CHEMOTHERAPEUTIC AGENTS**
ZUSAMMENSETZUNG ZUR POTENZIERUNG VON KREBS-CHEMOTHERAPEUTIKA
COMPOSITION DESTINES A POTENTIALISER DES AGENTS CHIMIOTHERAPIQUES CONTRE LE CANCER

(30) Priority: 01.09.2000 US 654377
(43) Date of publication of application: 05.03.2003
(73) Proprietor: The Ester C Company, Prescott AZ 86301 (US)
(72) Inventor: JARIWALLA, Raxit, J., Saratoga, CA 95070 (US)
(74) Representative: Brereton, Paul Arthur
(86) International application number: PCT/US2001/026455
(87) International publication number: WO 2002/020023

(56) References cited:
- EP-A- 0 344 880
- EP-A- 0 875 246
- WO-A-01/15692
- WO-A-90/12571
- WO-A-99/39580
- WO-A1-87/03481
- KURBACHER C M ET AL: "Ascorbic acid (vitamin C) improves the antineoplastic activity of doxorubicin, cisplatin, and paclitaxel in human breast carcinoma cells in vitro" CANCER LETTERS, NEW YORK, NY, US, vol. 103, 1996, pages 183-189, XP002251394 ISSN: 0304-3835
- PATENT ABSTRACTS OF JAPAN vol. 1997, no. 03, 31 March 1997 (1997-03-31) & JP 08 291075 A (SHOWA DENKO KK), 5 November 1996 (1996-11-05)

## Description

This Application is a Continuation-In-Part of and claims convention priority based on my copending USA Application, Serial Number 09/654,377, filed September 1, 2000.

### Background of the Invention

This invention relates to methods and compositions for potentiating the effectiveness of cancer chemotherapeutic agents.

In another respect, the invention pertains to such methods and compositions which permit one to increase the effectiveness of cancer chemotherapeutic agents, such that these agents can be used at lower dose rates, without sacrificing the efficacy of the agent.

In still another aspect, the invention concerns cancer chemotherapeutic compositions which include a combination of chemotherapeutic components which either cause cancer cell apoptosis or reduced proliferation rate or both.

Numerous cancer chemotherapeutic agents are known. In many cases, however, the administration of such agents at effective dose rates may cause undesirable side effects which severely limit their utility. Accordingly, it would be highly desirable to provide methods and compositions that would potentiate the chemotherapeutic effects of such agents, such that they could be effectively administered at lower dose rates, thereby reducing or eliminating such undesired side effects. Alternatively, such methods and compositions would make it possible to achieve greater chemotherapeutic effects from administration of given dose of a chemotherapeutic agent, with no increase in side effects.

### Brief Description of the Drawings

In the drawings:
Fig. 1 illustrates the effect of a representative vitamin C-derived furanone (FR) on normal liver cells and after three treatment applications with the furanone;
Fig. 2 illustrates the effect of the furanone on human hepatoma cells;
Fig. 3 illustrates the cell-death activity of the furanone (Preparation No. 1) in human melanoma cells and normal skin fibroblasts;
Fig. 4 illustrates the cell-death inducing activity of the furanone (Preparation No. 2) in human melanoma cells and normal skin fibroblasts;
Fig. 5 illustrates the effect on hepatoma of a dose of adriamycin (ADR) alone, ADR plus .5mM ascorbic acid (AA), ADR plus calcium ascorbate (CA) at .5mM AA equivalent following one and two applications;
Fig. 6 illustrates similar hepatoma suppression with ADR plus Ester-C (0.5mM AA equivalents);
Fig. 7 illustrates the influence of ADR with two different concentrations of the triple mixture (CA + CT + FR) following one application of ADR and three applications of the mixture;
Fig. 8 illustrates the relative effects of CA, EC and CA+CT+FR on ADR activity;
Figs. 9 and 10 illustrate the effects of the triple mix (CA+CT+FR) at various doses of ADR against human melanoma cells in two different tests;
Figs. 11 and 12 illustrate the effects on melanoma and hepatoma cells, respectively, after two sequential treatments with combinations containing different ratios of threonate to ascorbate, as measured by the BudR-based cell proliferation immunoassay;
Figures 13 and 14 compare the sensitivity of melanoma and hepatoma, respectively, after three treatments with mixtures containing various ratios of furanone to ascorbate, as assayed by the BudR cell proliferation ELISA;
Figures 15 and 16 illustrate the effect on melanoma and hepatoma, respectively, of triple mixtures containing increasing concentrations (from 1% to 7.5%) of furanone, in the above assay;
Figure 16A illustrates the effect on normal liver and hepatoma cells of the triple mixture (CA(92.5%)/CT(7.5%)/FR(7.5%) containing similar ascorbic-acid equivalents as in the double (CA + CT) mixture;
Fig. 17 illustrates the effect of furanone alone on the proliferation of the hepatoma cell line (SK-Hep-1);

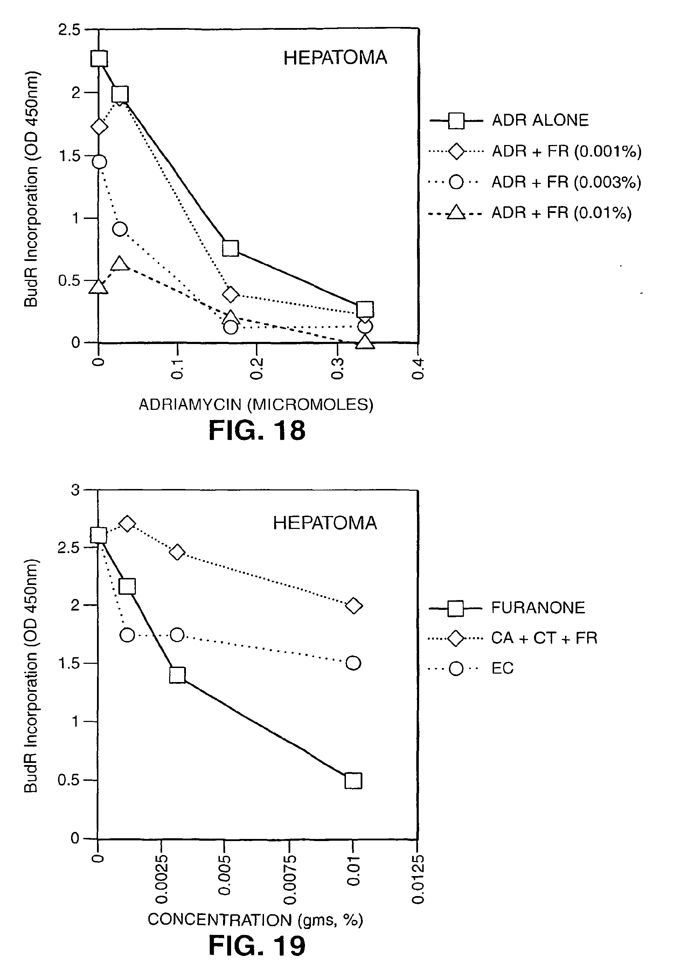

Fig. 18 illustrates the effect of furanone on the proliferation of hepatoma cells exposed to various concentrations of ADR;
Fig. 19 illustrates the effects of furanone alone, CA+CT+FR and Ester C (EC) on the proliferation of hepatoma cells;

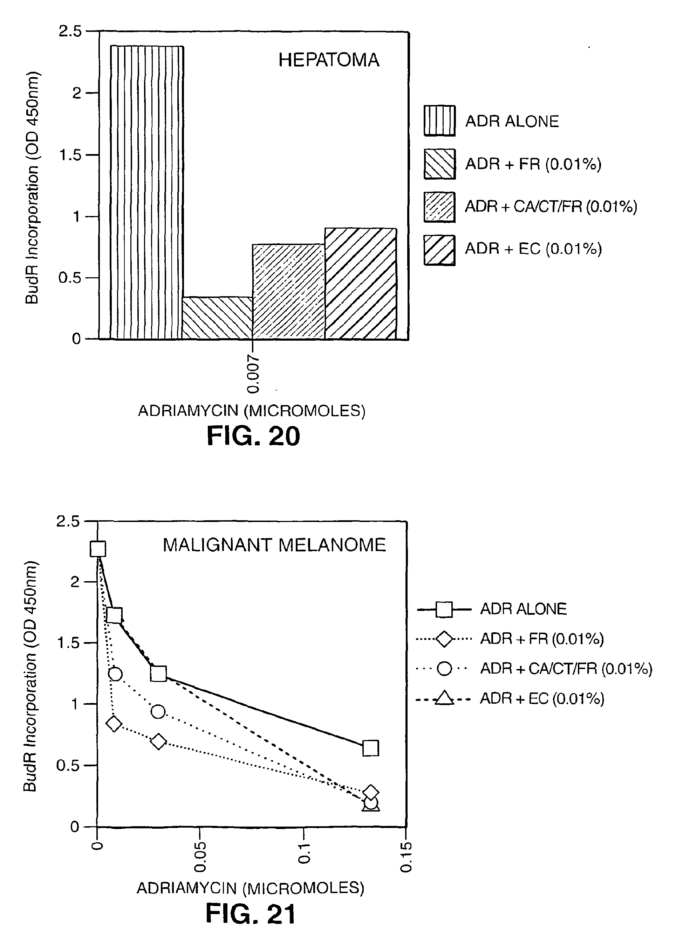

Fig. 20 illustrates the comparative activity on proliferation of hematoma cells of furanone, CA+CT+FR and EC in combination with ADR;
Fig. 21 illustrates the effects on proliferation of melanoma cells of furanone, CA+CT+FR and EC in combination with ADR at all concentrations of ADR.

### Brief Description of the Invention

I have now discovered that the chemotherapeutic effect of cancer chemotherapeutic agents (reduction in cancer cell proliferation rate and/or cancer cell apoptosis) is significantly potentiated by combining them with a potentiating component which is 4-hydroxy-5-methyl-3(2H)furanone.

By this technique, the same cancer chemotherapeutic effect is attained at significantly lower dose rates, in some cases at dose rates only about one-tenth of that required if the chemotherapeutic agent alone is administered. Further, I have discovered that such furanones potentiate the tumor cytotoxic and/or snti-prouferative effects of mineral ascorbates and mixtures of mineral ascorbates and other vitamin C metabolites.

In the presently preferred embodiment of the invention I employ a 4-hydroxy-5-methyl-3(2H) furanone as the potentiating component in combination with another cancer chemotherapeutic agent, to yield a composition having greater chemotherapeutic capability than either the furanone or the other chemotherapeutic agent alone. In tum, this allows the use of a lower dose of the other chemotherapeutic agent to achieve the same selective chemotherapuetic effects as would be obtained if the chemotherapeutic agent were employed alone, but at a higher dose.

### Detailed Description of the Invention

The furanone component of the compositions of the present invention can be synthesized by any suitable technique. Alternatively, one can employ a mixture of the furanone and plasma soluble mineral ascorbates available commercially under the registered trademark "Ester-C" from Inter-Cal Corporation, Prescott, Arizona, U.S.A.

According to the present invention there is provided a cancer chemotherapeutic composition comprising doxorubicin and 4-hydroxy-5-methyl-3(2H)furanone.

The methods of the present invention comprise administering a cancer chemotherapeutic dose, as a single dose or in repeated doses, of the above-described potentiated cancer chemotherapeutic compositions. Administration can be by any suitable technique normally used to administer cancer chemotherapeutic agents, e.g., orally or by parenteral injection.

### Illustrative Examples

The following examples are presented in order to illustrate the practice of the invention and the presently preferred embodiments thereof.

### EXAMPLE 1

This example illustrates the cell-death, cytotoxic potential of a representative furanone, 4-hydroxy-5-methyl-3(2H)-furanone, a vitamin C metabolite, against various human tumor -derived and normal cell types.

Four human tumor cell types, shown previously to be sensitive to combinations of mineral ascorbate plus metabolites are evaluated. These lines consist of: (i) a liver hepatoma (SK-Hep-1), (ii) a malignant melanoma (Malme-3M), (iii) a neurobastoma (SK-N-MC) and (iv) a colon carcinoma (T84). Non-malignant cell types consist of: normal human liver (WRL 68), normal skin fibroblasts (Malme-3) and normal retinal epithelial cells (ARPE-19). The tumor-derived lines, normal liver and skin fibroblasts are obtained from the American Type Culture Collection (ATCC). The ARPE-19 cell line is a gift from a colleague at California Institute for Medical Research. Cells are grown and maintained in culture, in a humidified incubator of 5% CO₂/95% air.

Samples of 4-hydroxy-5-methyl-3(2H)-furanone, a highly purified synthesis product are provided by Inter-Cal Corporation, Prescott, Arizona, in two batches (Preparations 1 and 2) of frozen product. The samples are stored frozen immediately after receipt and when not in use. Master stocks (1% w/v) of the furanone are prepared in sterile water and stored frozen for duration of each experiment. On the day of treatment, master stocks are serially diluted in sterile water to obtain 10X strength working stocks.

Subconfluent monolayers of 1-2 old, adherent cultures (seeded at a density of 10,000 cells per well in a 96-well plate) are exposed to 0.1 volume of serial dilutions (10X concentrated solutions) of the furanone and the plate is returned to the incubator. Furanone treatment (second application) is repeated after 48 hrs by direct addition of respective 10X solution without change of culture medium. After further incubation for 48 hrs., the culture medium is replaced with fresh growth medium, followed by a third application of the corresponding furanone concentration. Control wells receive an equal volume of sterile water (in place of furanone solution) and are handled identically. After final incubation of 48 hrs. (following third treatment), cells lysates are prepared by extraction of the monolayer with 0.2 ml of lysis solution followed by low-speed centrifugation (2500 rpm, 10 min). The supernatant (cytoplasmic lysate) is saved and in aliquot (20 microliters) was assayed for the presence of histone-DNA complexes (nucleosomal fragments) by photometric detection (absorbance at 405 - 410 nm) using cell death detection Elisa assay (Roche Diagnostics).

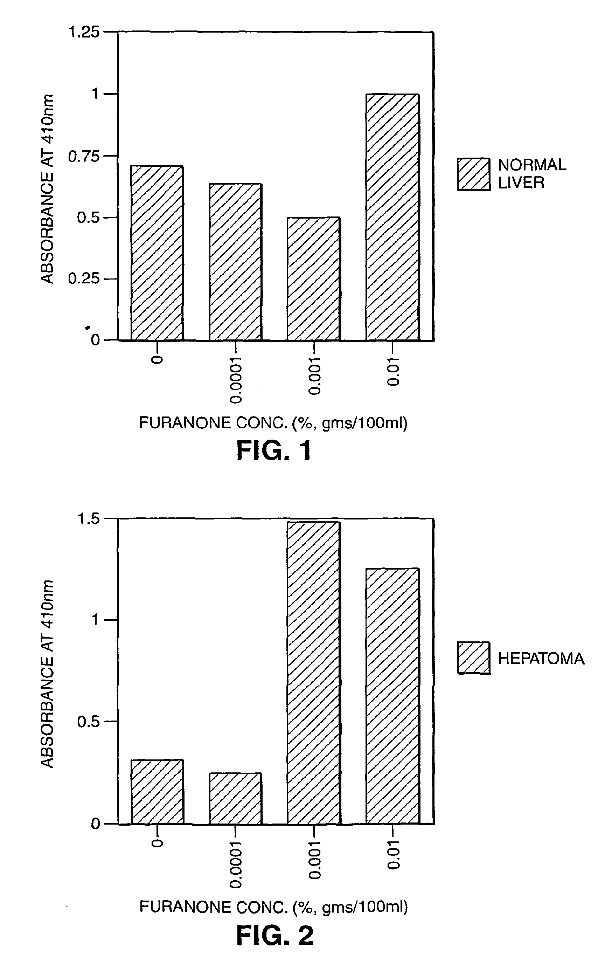

The effects of the furanone on normal human liver and hepatoma cells after three sequential treatments are shown in Figures 1 and 2. In the concentration range (0.01 - 0.0001%) evaluated, the compound produces a maximal change in cell-death rate of less than 0.3 logs in normal liver cells (Fig. 1). In contrast, sharp rises in cell death are seen in hepatoma cells treated with furanone concentrations in the 0.001 - 0.01% range (Fig. 2), which corresponds to a molarity range of 0.08 - 0.88 mM furanone. This effect in hepatoma cells declines with increasing furanone concentration (approaching 0.1 %), possibly due to cell loss occurring from change in pH of the culture medium at the highest dose (0.1% furanone, data not shown).

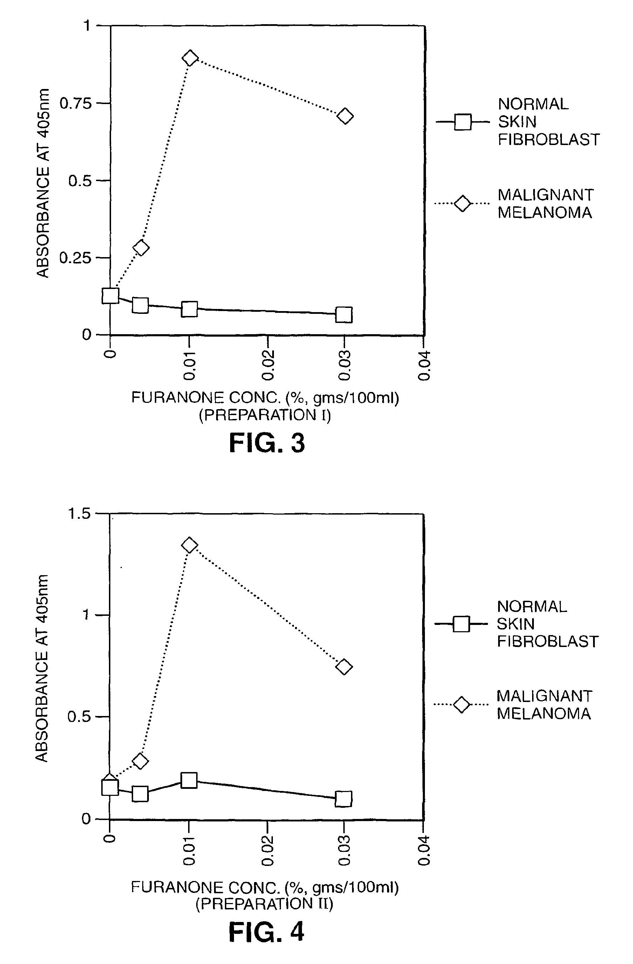

Figures 3 and 4 display the effects of two different preparations of the furanone on malignant melanoma (Malme-3M) and its normal cellular counterpart, human skin fibroblasts (Malme-3). A similar cell-death inducing specificity to that seen in hepatoma cells is observed in malignant melanoma. Thus, both furanone preparations induced dose-dependent death of the melanoma line (from 5 to 10 fold increase in apoptosis) relative to an unremarkable effect on normal skin fibroblasts (<0.3 log increase in apoptosis). The minimal concentration required to produce 2-fold change in apoptosis rate is slightly higher for melanoma cells (0.003% furanone) than that for hepatoma cells (<0.001% furanone).

In contrast to the effects on hepatoma and melanoma cell types, furanone treatment of colon carcinoma cells produces <0.5 log change in rate of cellular apoptosis (over that of untreated control) when evaluated in a comparable concentration range (up to a max. of 0.01 % furanone). At lower concentration ranges (up to max of 0.003% in ARPE-19 and 0.001% in SK-N-MC), furanone treatment does not elicit significant changes in death rates of normal epithelial and neuroblastoma cells.

Thus, treatment of malignant and non-malignant human hepatoma and melanoma cell lines by 4-hydroxy-5-methyl-3(2H)-furanone, a metabolite of vitamin C, shows preferential cytotoxicity of the compound towards malignant cells as indicated by tumor-specific induction of cell death.

### EXAMPLE 2

The following procedures are carried out to illustrate the activity of calcium ascorbate (CA), calcium ascorbate-calcium threonate compositions (CA+CT), calcium ascorbate-threonate-furanone compositions (CA+CT+FR) and a commercially available calcium ascorbate-metabolites-furanone composition (EC) available under the trademark Ester-C® from Inter-Cal Corporation, Prescott, Arizona, USA, alone and in combination with a typical cancer chemotherapuetic agent, adriamycin (doxorubicin).

Calcium ascorbate (CA), calcium threonate (CT), 4-hydroxy-5-methyl-3(2H)-furanone (FR) and Ester-C (EC) are obtained from Inter-Cal Corporation, Prescott, Arizona, USA. Tissue culture grade Ascorbic acid (AA) is from Sigma Chemical Company, St. Louis, MO, USA. The composition of calcium ascorbate (calcium di-(L-ascorbate) dihydrate) is made by sequentially dissolving the salt in sterile water to obtain master-stock solutions of equal strength (1% w/v). Aqueous solution of calcium ascorbate plus calcium threonate plus 4-hydroxy-5-methyl-3(2H)-furanone (CA/CT/FR) is prepared at ratio of 85/7.5/7.5. For comparative analysis, stock solutions containing identical ascorbic acid (AA) equivalents are used. All master stocks are stored at room temperature during the course of these procedures. From these, 10X strength working stocks are prepared by serial dilution on the day of use and are applied to cells in microtiter plates as described below.

Adriamycin (doxorubicin) is obtained from Sigma Chemical Company (St. Louis, MO). Stock solutions are made by dissolving adriamycin (ADR) in sterile water and DTIC in water or dilute acetic acid solution (0.01N). Stocks are stored frozen at -10C and thawed prior to use. Serial dilutions are made in sterile water to obtain 10X strength solutions and one tenth volume of this is applied to cells at the time of treatment.

Target human tumor-derived lines consist of SK-Hep-1, a liver adenocarcinoma (hematoma) and Malme-3M, a malignant melanoma. All seed cultures are obtained from ATCC, Manassas, VA, USA and are propagated/maintained in serum-containing growth medium in a humidified incubator of 5% CO₂/95% air. SK-Hep-1 cells are grown in Eagle's minimum essential medium (EMEM) in Earle's salts plus non-essential amino acids supplemented with 10% FBS plus antibiotics and Malme-3M cells are cultured in McCoy's medium supplemented with 15% fetal bovine serum (FBS) plus antibiotics.

For treatment, exponentially growing cells are seeded at a density of 1 x 10⁴ cells per well of 96-well microtiter plate in 0.1 - 0.2 ml of growth medium. After overnight cell attachment at 37 C, duplicate wells are exposed to various treatments by direct application of one-tenth volume of 10x strength solution. For treatments involving chemotherapeutic agent, one-tenth volume of serial dilutions of adriamycin (0.0006 - 3.0 micromolar) are applied to wells followed by application of ascorbate-containing composition (adjuvant) or sterile water (control). The adjuvant treatment is repeated every other day or on the third day (as applicable) without change of growth medium. Untreated controls receive an equivalent amount of sterile water. After 2 - 3 treatments with adjuvant, cells are labeled with BudR as described below. (In some experiments, wells are refed an equal volume of growth medium prior to additional of BudR. This gives a higher amount of BrdU incorporation into DNA relative to no refluiding).

For DNA labeling, the following procedure is adapted and modified from the Cell Proliferation ELISA, BudR (Colorimetric) assay of Roche Diagnostics, Indianapolis, IN, USA: Each treated well is exposed to one-tenth volume of 10X BrdU-containing medium and reincubated at 37 C overnight (15 - 20 hrs.). One set of control wells is exposed to medium only (no BudR) to obtain an absorbance-control (substrate only) blank for the assay. After the labeling period, the culture medium is removed and the cells are fixed and DNA denatured in one step by adding FixDenat (30 min at RT). Then, FixDenat is removed, the anti-BrdU-peroxidase reagent is added and incubation continues for 90 min at RT. After removal of the latter, the plate is washed 3 times, followed by addition of substrate solution. The plate is kept at RT until color development became sufficient for photometric detection (5-10 min). At this time, reaction is stopped by addition to each well of 25 microliter 1M sulfuric acid followed by mixing for approximately 1 min on the shaker. The absorbance of the samples is measured in an ELISA reader at 450 nm, within 5 minutes after adding stop solution. The OD₄₅₀ₙₘ is a measure of the level of BudR incorporation into DNA, which is proportional to rate of cell proliferation. For data analysis, the mean of the absorbance is plotted against concentration of chemotherapeutic drug for each treatment to obtain a dose-response relationship of the anti-proliferative effect.

### Influence of Ascorbate, Ester-C and Triple Ascorbate plus Metabolites Combination on Anti-Neoplastic Activity of Adriamycin Against Hepatoma Cells

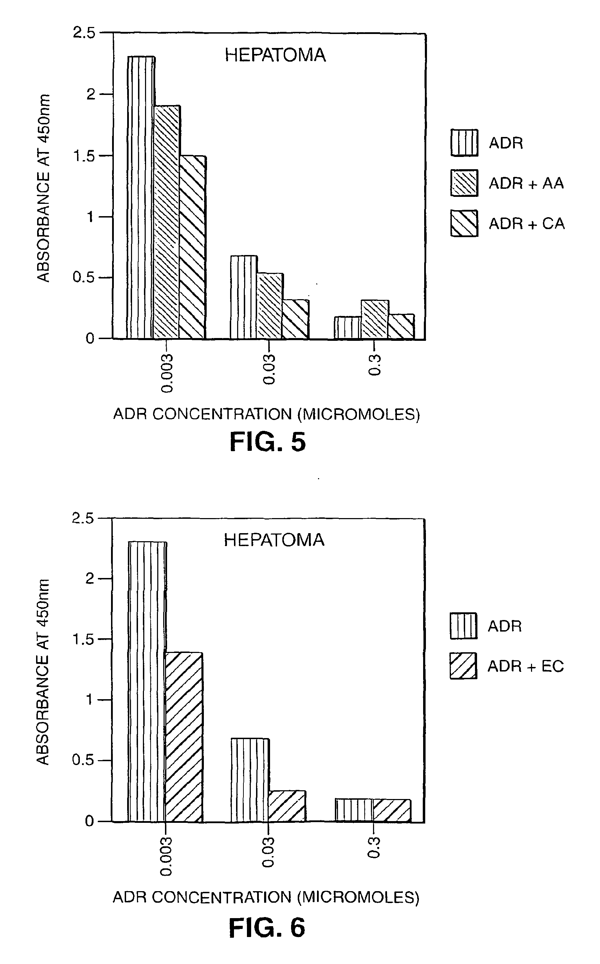

Fig. 5 shows the effects of a dose of adriamycin (ADR) alone, ADR plus 0.5mM ascorbic acid and ADR plus calcium ascorbate (0.5mM AA equivalents) following one application of ADR and two applications of adjuvant (i.e. AA or CA). Note that combined treatment with adjuvant (AA or CA) gives greater suppression of hepatoma proliferation than ADR alone. The enhancing effect is prominent at low to moderate doses of ADR (0.003 - 0.03 micromolar) compared to higher ADR dose (0.3 micromolar). Calcium ascorbate is more effective than ascorbic acid. Similar type of hepatoma suppression is seen with ADR plus Ester-C (containing 0.5 mM AA equivalents) in the same test.

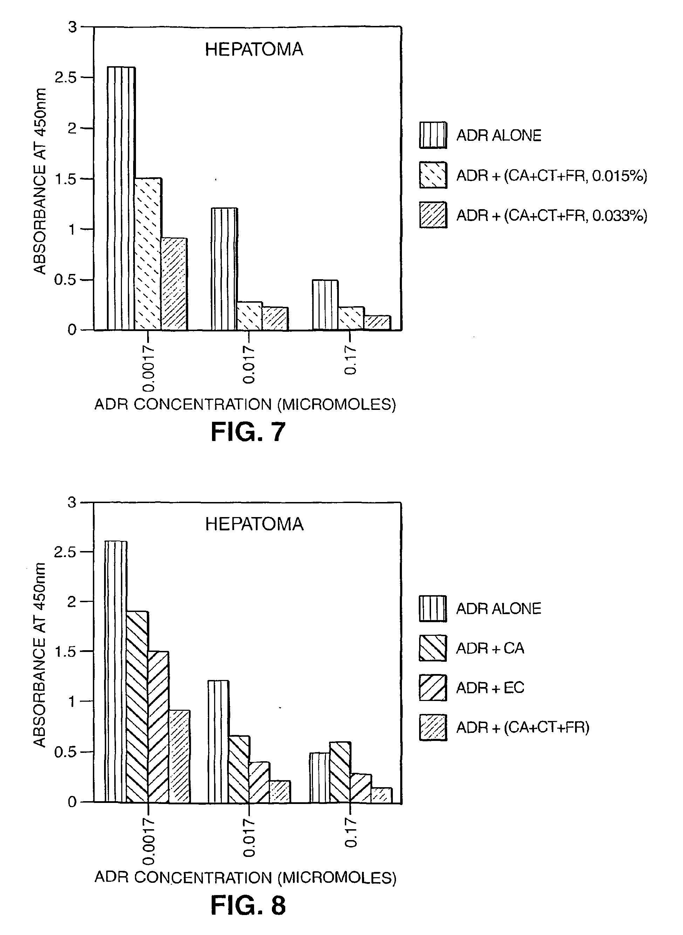

In another procedure, the effects of a triple ascorbate metabolites combination (CA+CT+FR @ ratio of 85:7.5:7.5) on ADR activity is evaluated in parallel with CA or EC. Fig. 7 shows the influence of 2 different concentrations (0.015% and 0.033%) of the triple mixture following one application of ADR and three applications of the mix. As can be seen from the graph, greater proliferation of hepatoma cells occurs following ADR + triple combination compared to ADR alone. Again, the enhancing effect is more prominent at low to moderate doses (0.0017 - 0.017 micromolar) of ADR than the higher dose (0.17 micromolar) ADR tested.

Fig. 8 shows the relative effects of 0.033% CA, EC and CA+CT+FR on ADR activity in the same experiment. All three ascorbate-containing compositions consistently improve the anti-proliferation activity of low doses of ADR, with EC and the triple ascorbate plus metabolites mix producing greater improvement than CA. Most notably, both EC and CA+CT+FR when used in combination with 0.017 micromolar ADR gives about the same or slightly better anti-neoplastic effect as compared to a 10-fold higher dose (0.17 micromolar) of ADR alone.

### Effect of Triple Ascorbate plus Metabolites Composition on Anti-Proliferative Activity of Adriamycin in Melanoma Cells

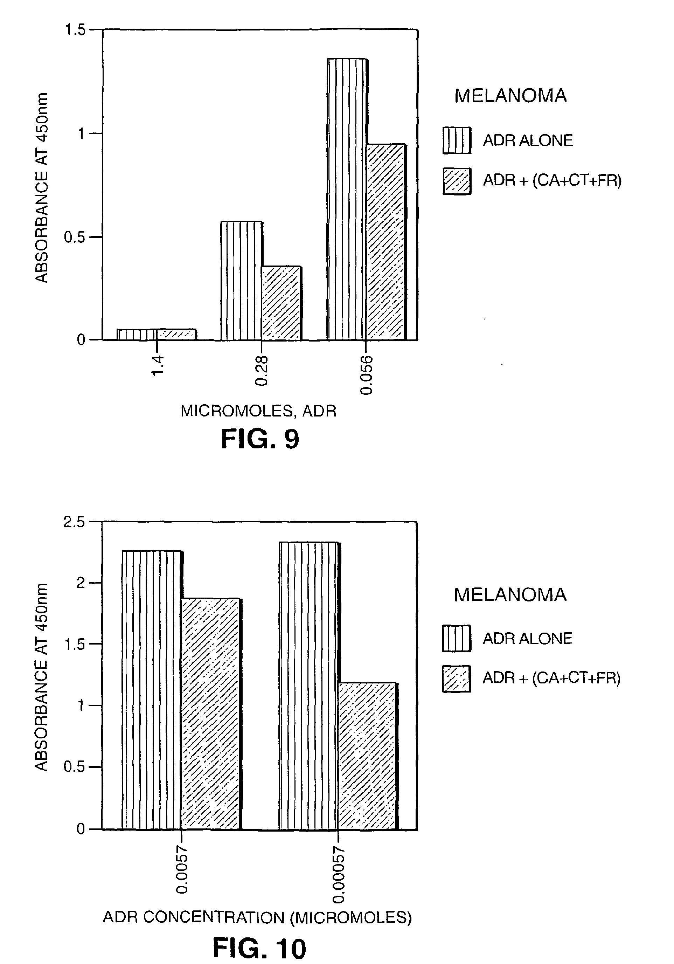

Figures 9 and 10 show the effects of the triple mix (CA+CT+FR, at 0.033%) plus various doses of ADR against human melanoma cells, from two different tests. In both tests, the triple mix potentiates the anti-neoplastic activity of ADR preferentially at low to moderate doses of the chemotherapeutic drug.

Thus, ascorbate-containing compositions improve the anti-neoplastic activity of cancer chemotherapeutic agents (adriamycin), against both hepatoma and melanoma-derived cell lines as evaluated using a cell-proliferation immunoassay. The enhancing effect is most prominent at low to moderate doses of the chemotherapeutic drug. Compositions containing ascorbate plus metabolites are more effective in enhancing adriamycin activity than ascorbate alone. Triple mixtures containing calcium ascorbate, calcium threonate and furanone (at ratio of 85:7.5:7.5) when combined with low-dose adriamycin suppress tumor cell proliferation at a level similar to or slightly better than a 10-fold higher dose or adriamycin alone. These results indicate the use of ascorbate plus metabolites in combination with low-dose chemotherapy with reduction of potential drug-associated toxicity.

### Example 3

This example illustrates the activity of various ascorbate plus metabolite mixtures against human tumor-derived melanoma and hepatoma cell lines, using cell-proliferation immunoassay based on BrdU incorporation during DNA synthesis.

Aqueous solutions of the following mixtures are freshly prepared for use in each experiment:
Calcium ascorbate plus calcium threonate (CA/CT) at ratios of:
   99/1 (standard mix),
   97.2/2.5
   95/5
   92.5/7.5 and
   90/10
Calcium ascorbate plus 4-hydroxy-5-methyl-3(2H)-furanone (CA/FR) at ratios of:
   99.9/0.1 (standard mix)]
   99/1
   97.2/2.5
   95/5 and
   92.5/7.5
Calcium ascorbate plus calcium threonate plus 4-hydroxy-5-methyl-3(2H)-furanone (CA/CT/FR) at ratios of:
   91.5/7.5/1
   90/7.5/2.5
   90/7.5/2.5
   88.5/7.5/5
   85/7.5/7.5
   90/10/1 and
   80/10/10

Calcium ascorbate, calcium threonate and 4-hydroxy-5-methyl-3(2H)-furanone which is obtained from Inter-Cal Corporation, Prescott, Arizona. The composition of calcium ascorbate (Calcium di-(L-ascorbate) dihydrate) is L-ascorbic acid (82.15%), calcium (9.45%) and water (8.44%).

Aqueous mixtures are made by sequentially dissolving individual components in sterile water to obtain master-stock solutions of equal strength (1% w/v) for comparative analysis. In some cases, stock solutions of 1.08% strength are prepared to obtain comparable ascorbic-acid equivalents between double and triple combination mixtures. All master stocks are stored at room temperature during the course of these experiments. From these, 10X strength working stocks are prepared by serial dilution on the day of use and applied to cells in micro titer plates as described below. The standard mixes contain constituent metabolites at ratios reflecting those in "Ester-C" brand Calcium Ascorbate are obtained from Inter-Cal Corporation, Prescott, Arizona..

Target human tumor-derived or normal lines consist of Malme-3M, a malignant melanoma, SK-Hep-1, a liver adenocarcinoma (hepatoma) and WRL 68 cells, a line of normal liver cells. All seed cultures are obtained from ATCC (Manassas, VA) and are propagated/maintained in serum-containing growth medium in a humidified incubator of 5% CO₂/95% air. Malme-3M cells are cultured in McCoy's medium supplemented with 15% fetal bovine serum (FBS) plus antibiotics and SK-Hep-1 and CL-48 lines are grown in Eagles' minimum essential medium (EMEM) in Earle's salts plus non-essential amino acids supplemented with 10% FBS plus antibiotics.

The following procedure is adapted and modified from the Cell Proliferation ELISA, BudR (Colorimetric) assay of Roche Diagnostics (Indianapolis, IN).

For treatment, exponentially growing cells are seeded at a density of 1 x 10⁴ cells per well of 96-well micro titer plate in 0.1 - 0.2 ml of growth medium. After overnight cell attachment at 37 C, duplicate wells are exposed to serial dilutions of ascorbate/metabolite mixture by direct application of one-tenth volume of 10x strength solution. The treatment is repeated daily by application of respective ascorbate/metabolite mixture without change of growth medium. Untreated controls receive an equivalent amount of sterile water. After 2-3 daily treatments, cells are labeled with BudR as described below. (In some experiments, wells are re-fed an equal volume of growth medium prior to addition of BudR. The gave a higher amount of BrdU incorporation into DNA relative to no refluiding).

For DNA labeling, each treated well is exposed to one-tenth volume of 10X BrdU-containing medium and reincubated at 37 C overnight (15-20 hrs). One set of control wells is exposed to medium only (no BudR) to obtain an absorbance-control (substrate only) blank for the assay. After the labeling period, the culture medium is removed and the cells are fixed and DNA denatured in one step by adding FixDenat (30 min at RT). Then, FixDenat is removed, the anti-BrdU-peroxidase reagent is added and incubation continued for 90 min at RT. After removal of the latter, the plate is washed 3 times, followed by addition of substrate solution. The plate is kept at RT until color development becomes sufficient for photometric detection (5-10 min). At that time, reaction is stopped by addition of 25 microliter 1M sulfuric acid to each well and the plate is placed for approximately one minute on the shaker. The absorbance of the samples is measured in an ELISA reader at 450 nm, within 5 min after adding stop solution. The OD at 450 nm is a measure of the level of BudR incorporation into DNA, which is proportional to rate of cell proliferation. For data analysis, the mean of the absorbance is plotted against concentration of ascorbate/metabolite mixture to obtain a dose-response relationship of the anti-proliferative effect.

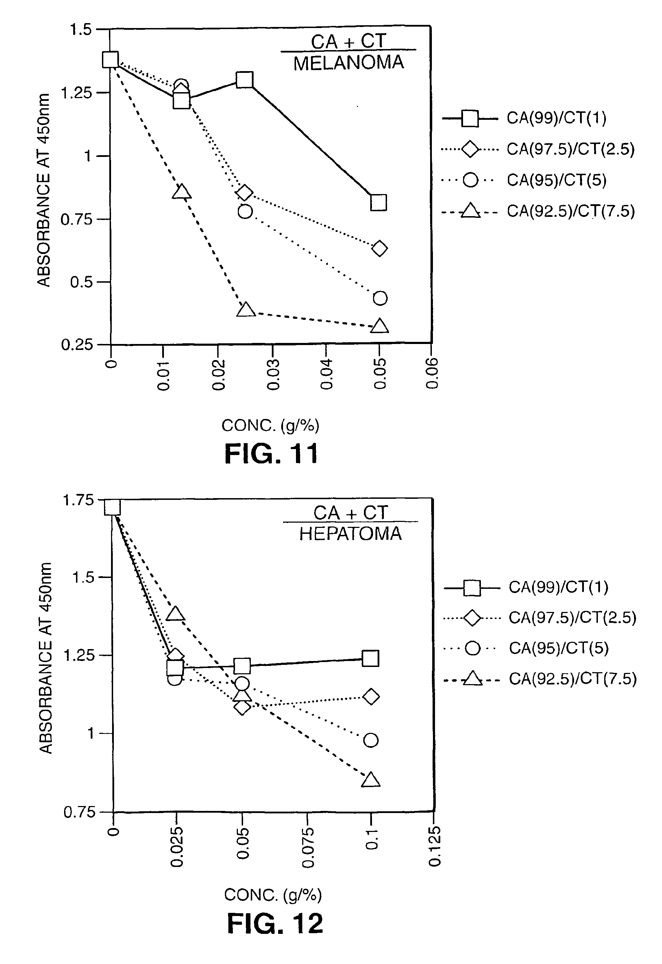

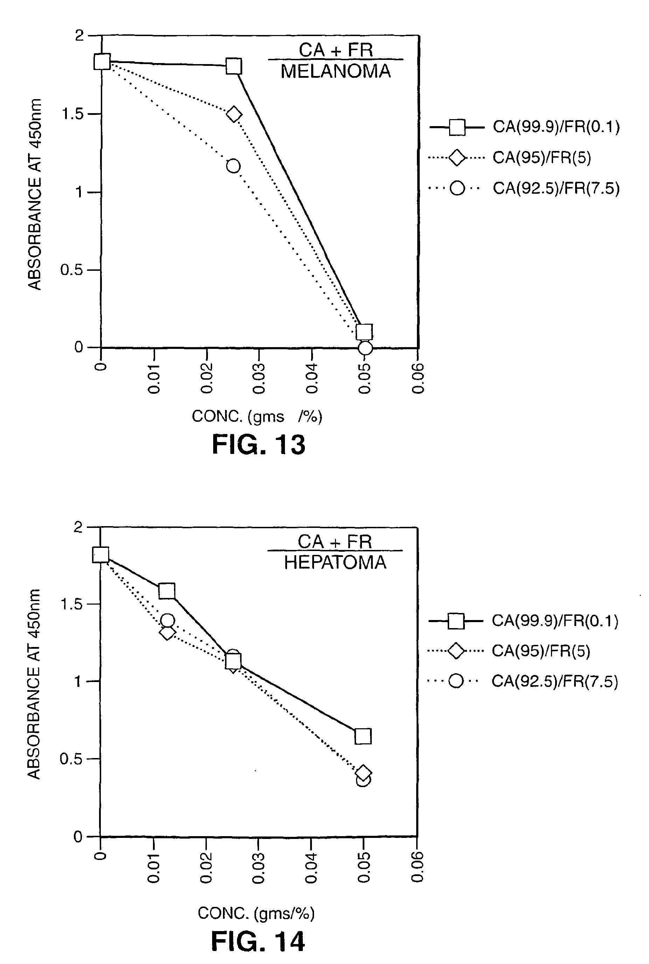

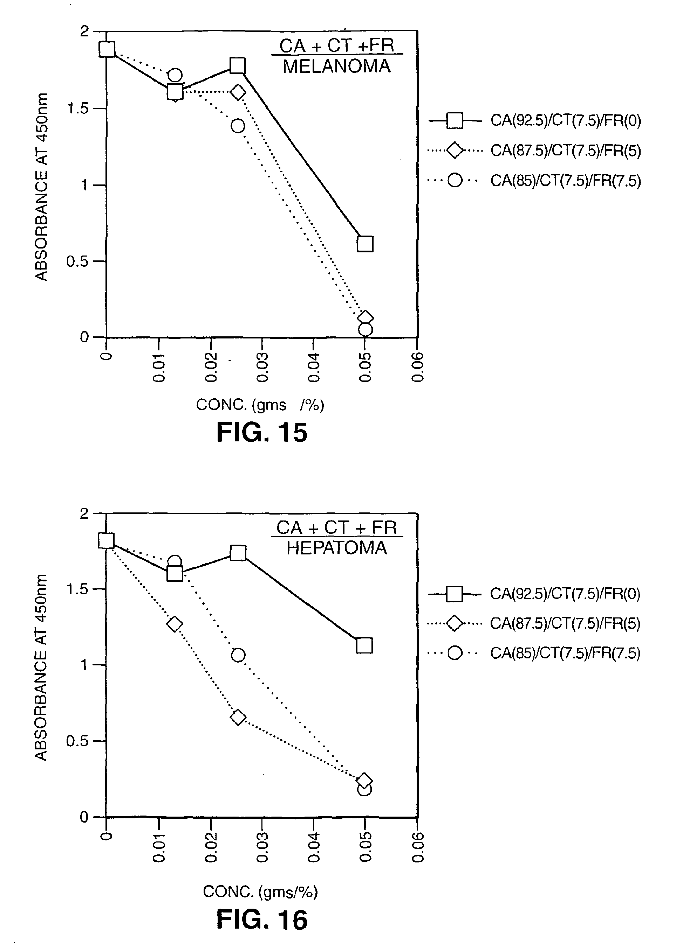

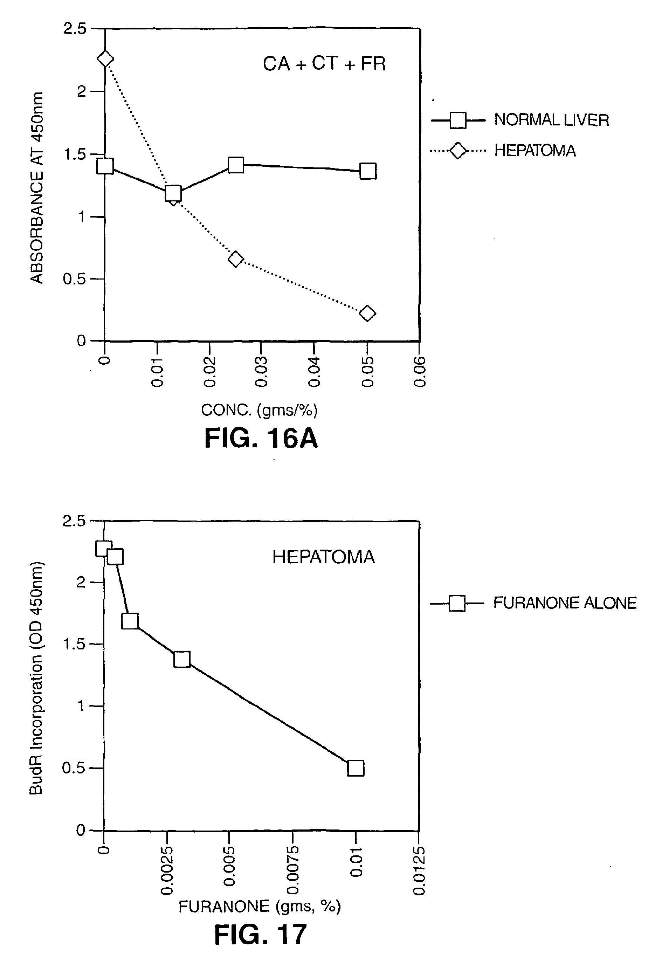

The results of such experiments, shown graphically in Figs. 11 - 16A, illustrate that:
- Mixtures containing ascorbate, threonate and/or furanone at various ratios elicit tumor-specific suppression of cell proliferation in melanoma and hepatoma cell types.
- Increasing the ratio of calcium threonate from 1% to 7.5% in the CA + CT mixture, enhances the relative anti-tumor activity of the double combination in both tumor cell types.
- Addition of furanone to calcium ascorbate enhances anti-proliferative activity in melanoma cells, with the double CA/FR combination (92.5%/7.5% ratio) displaying higher activity than the simulated CA/FR mixture (99.9%/0.1% ratio). There is approximately a 9-fold difference between these two mixtures at the 0.05% dose (OD of 0.008 vs 0.088).
- Addition of furanone to the CA +CT double mixture enhances its activity in both tumor cell types, with the triple CA/CT/FR combination (85/7.5/7.5 ratio) giving optimal activity. The enhancing effect of furanone addition against hepatoma is more prominent in the triple mix than in the double (CA +FR) combination. Further increases in the relative level of FR and CT to 10% do not improve the anti-tumor activity of the triple combination (data not shown).
- In contrast to the effects seen in tumor cells, the triple mixture CA/CT/FR does not suppress proliferation in normal liver cells, even when tested at (relatively higher) ascorbic acid equivalents (92.5/7.5/7.5) comparable to that contained in the double CA + CT mixture (92.5/7.5).

Thus, double mixtures of calcium ascorbate plus calcium threonate (CA + CT) or calcium ascorbate plus furanone (CA + FR) at ratios of 92.5 : 7.5 and triple mixtures of the three components (CA + CT + FR) at ratio of 85 : 7.5 : 7.5 manifest optimal anti-proliferative activity against malignant melanoma and hepatoma cell types in a rapid screening immunoassay. Such mixtures allow anti-tumor effects to be observed at lower ascorbic-acid equivalents than that contained in simulated standards.

### Example 4

This example illustrates the activity of the representative furanone, 4-hydroxy 5-methyl-3(2H)-furanone, alone and the furanone plus adriamycin against human hepatoma and melanoma cells, using cell-proliferation immunoassay based on BrdU incorporation into cellular DNA.

A newly synthesized batch of 4-hydroxy-5-methyl-3(2H)-furanone (FR) is provided by Inter-Cal Corp., Prescott, Arizona. Master stock solutions (0.1% or 0.75% strength) are made by dissolving desired amount of furanone in sterile water and stored frozen (at - 10 C) until use. Calcium ascorbate (CA), calcium threonate (CT), and Ester-C (EC) is also obtained from Inter-Cal. The composition of calcium ascorbate (calcium di-)L-ascorbate) dihydrate is L-ascorbic acid (82.15%), calcium (9.45%) and water (8.44%). Aqueous mixtures of ascorbate plus metabolites are prepared by sequentially dissolving individual components in sterile water to obtain master-stock solution of desired strength. A triple mix of calcium ascorbate plus calcium threonate plus 4-hydroxy-5-methyl-3(2H)-furanone (CA/CT/FR) is prepared at ratio of 85/7.5/7.5 or 87.5/7.5/5.0. Master stocks are kept at room temperature during the course of the experiment. From these, 10X strength working stocks is prepared by serial dilution on the day of use and applied to cells in microtiter plates as described below.

Adriamycin (doxorubicin) is obtained from Sigma Chemical Company (St. Louis, MO). Stock solutions are made by dissolving adriamycin (ADR) in sterile water. Stocks are stored frozen at -10 C and thawed prior to use. Serial dilutions are made in sterile water to obtain 10X strength solutions and 0.1 volume of this is applied to cell at the time of treatment.

Target human tumor-derived lines consist of SK-Hep-1, a liver adenocarcinoma (hepatoma) and Malme-3M, a malignant melanoma. Seed cultures are obtained from ATCC (Manassas, VA) and propagated/maintained in serum-containing growth medium in a humidified incubator of 5% CO₂/95% air. SK-Hep-1 cells are grown in Eagles' minimum essential medium (EMEM) containing Earles' salts plus non-essential amino acids supplemented with 10% fetal bovine serum (FBS) plus antibiotics and Malme-3M cells are cultured in McCoy's medium supplemented with 15% FBS plus antibiotics.

For treatment, exponentially growing cells are seeded at a density of 1 x 10⁴ cells per well in a 96-well microtiter plate in 0.1 - 0.2 ml of growth medium. After overnight cells attachment at 37 C, duplicate wells are exposed to various treatments by direct application of one-tenth volume of 10x strength solution. For treatments involving chemotherapeutic agent, one-tenth volume of serial dilutions of adriamycin (in the indicate dosage range) are applied to wells followed by application of adjuvant (i.e. furanone or ascorbate plus metabolites). The treatment with adjuvant is repeated daily or every other day (as applicable) without change of growth medium. Untreated controls received an equivalent amount of sterile water. After - 3 treatments with adjuvant, cells are labeled overnight with bromo-deoxyuridine (BudR), as described below. (In some experiments, wells are re-fed an equal volume of growth medium prior to addition of BudR. This gave a higher amount of BrdU incorporation into DNA relative to no refluiding).

For DNA labeling, the following procedure is adapted and modified from the Cell Proliferation ELISA, BudR (Colorimetric) assay of Roche Diagnostics (Indianapolis, IN). Each treated well is exposed to one-tenth volume of 10X BrdU-containing medium and reincubated at 37 C overnight (15-20 hrs). One set of control wells is exposed to medium only (no BudR) to obtain an absorbance-control (substrate only) blank for the assay. After the labeling period, the culture medium is removed and the cells are fixed and DNA denatured in one step by adding FixDenat (30 min at RT). Then, FixDenat is removed, the anti-BrdU-peroxidase reagent added and incubation continued for 90 minutes at room temperature. After removal of the latter, the plate is washed 3 times, followed by addition of substrate solution. The plate is kept at room temperature until color development became sufficient for photometric detection (5-10 min). At this time, reaction is stopped by addition to each well of 25 microliter 1M sulfuric acid followed by mixing for approx. 1 min on the shaker. The absorbance of the samples is measured in an ELISA reader at 450 nm, within 5 min after adding stop solution. The OD₄₅₀ₙₘ is a measure of the level of BudR incorporation into DNA, which is proportional to rate of cell proliferation. For data analysis, the mean of the absorbance is plotted against concentration of adjuvant or chemotherapeutic drug from each treatment to obtain a dose-response relationship of the anti-proliferative effect.

The concentration range of the tests (0.003-0.01%), a dose-dependent reduction of hepatoma proliferation is seen, with 0.01 % furanone producing ~ 4.6 fold reduction in cellular DNA synthesis compared to untreated control. A similar dose-depended profile is obtained in 3 independent experiments.

ADR along produces dose-dependent inhibition of hepatoma proliferation in the range of the tests (0.02 - 0.33 micromoles). When combined with furanone (0.001 - 0.01 %), the combination confers greater inhibition, producing from approximately < 2 to 22 fold enhancement of hepatoma inhibition over that obtained with corresponding dose of ADR alone. (From two independent experiments, the minimal dose of furanone required to produce 2-fold enhancement of ADR inhibition is ~ 0.0016%, which corresponds approximately to the absolute concentration of furanone in a 1% Ester-C solution.) These experiments define the range of furanone concentration which is to be used in subsequent experiments of comparative analysis.

In the concentration range tested (0.001 - 0.01 %), furanone is more effective in inhibiting hepatoma proliferation than either the triple ascorbate/metabolite mix or Ester-C® when compared at the same absolute concentration in aqueous solution.

At the minimal dose of ADR (0.007 micromolar) and maximal dose of adjuvant (0.01%), furanone enhances the inhibitory effect of ADR by 7-fold compared to 3.1 and 2.7 fold enhancements one obtains respectively with the triple mix and EC.

Furanone (at 0.1 %) consistently enhances the inhibitory effect of ADR at all concentrations of the chemotherapeutic drug. In contrast, whereas the triple mix and EC gives comparable enhancement at moderate doses of ADR (0.136 micromolar), furanone performs better than either composition at low doses of ADR (0.005 - 0.27 micromolar).

Thus, furanone alone is capable of inhibiting cell proliferation in both hepatoma and melanoma cell lines. Additionally, furanone can function as an adjuvant in combination with a chemotherapeutic drug (adriamycin), enhancing its inhibitor effects in combinatorial experiments. On a weight-to-weight basis, the anti-proliferative and adjuvant (synergistic) effects of furanone are higher than those of triple ascorbate/metabolite mix and Ester-C, indicating that the chemical structure of furanone may contain the active component responsible for the anti-tumor effects of compositions containing ascorbate and its metabolites.

Having described my invention in such terms as to enable those skilled in the art to understand and practice it and, having identified the presently preferred embodiments there, I CLAIM:

## Claims

1. A cancer chemotherapeutic composition comprising doxorubicin and 4-hydroxy-5-methyl-3(2H)furanone.

2. A composition according to any preceding claim for the treatment of cancer.

3. Use of a composition according to any of claims 1 to 3 in the manufacture of a medicament for the treatment of cancer.

## Patentansprüche

1. Krebs-chemotherapeutische Zusammensetzung, umfassend Doxorubicin und 4-Hydroxy-5-methyl-3(2H)-furanon.

2. Zusammensetzung nach einem der vorangehenden Ansprüche für die Behandlung von Krebs.

3. Verwendung einer Zusammensetzung nach einem der Ansprüche 1 bis 3 bei der Herstellung eines Arzneimittels zur Behandlung von Krebs.

## Revendications

1. Composition chimiothérapeutique contre le cancer composée de doxorubicine et de 4-hydroxy-5-méthyle-3(2H)furanone.

2. Composition selon toute revendication précédente pour le traitement du cancer.

3. Utilisation d'une composition selon l'une quelconque des revendications 1 à 3 dans la fabrication d'un médicament pour le traitement du cancer.
